# EUROPÄISCHE PATENTSCHRIFT

(11) **EP 2 453 002 B1**
(45) Veröffentlichungstag und Bekanntmachung des Hinweises auf die Patenterteilung: **10.10.2012**
(21) Anmeldenummer: 10190810.1
(22) Anmeldetag: 11.11.2010
(51) Int. Cl.: C11C 5/00, A61F 11/00

(54) **Ohrkerze**
Ear candle
Bougie d'oreille

(43) Veröffentlichungstag der Anmeldung: 16.05.2012
(73) Patentinhaber: Leschik, Udo, 35630 Ehringshausen (DE)
(72) Erfinder: Leschik, Udo, 35630 Ehringshausen (DE)
(74) Vertreter: Bauer Vorberg Kayser Patentanwälte

(56) Entgegenhaltungen:
- EP-A1- 0 908 161
- DE-A1-102006 004 138
- FR-A1- 2 768 924

## Beschreibung

Die Erfindung betrifft eine Ohrkerze mit einem luftdurchlässigen Rückhalteelement, welches innerhalb der Ohrkerze angeordnet ist.

Zum Stand der Technik (DE 10 2006 004 138 Al) gehört eine Ohrkerze mit integriertem Rückhalteelement, bei dem an einem rückwärtigen Ende, das heißt einem ohrseitigen Ende der Ohrkerze, das Rückhalteelement innerhalb der Ohrkerze angeordnet ist.

Es handelt sich bei diesem Rückhalteelement um ein luftdurchlässiges Rückhalteelement, welches eine Querschnittsfläche aufweist, welche spitzkegelig oder stumpfkegelig oder kuppelförmig oder schrägdachförmig oder pyramidenförmig oder hohlzylindrisch ist, wobei die Spitze oder der Stumpf oder die Krümmung dieses Rückhalteelementes in Richtung eines frontwärtigen Endes der Ohrkerze ausgerichtet ist.

Die Ohrkerze besteht im Wesentlichen aus einem mit Wachs getränkten Baumwollstoff. Als Wachs kann ein natürliches Wachs wie zum Beispiel Bienenwachs verwendet werden. Es ist auch möglich, synthetische Wachse wie Stearin oder Paraffin zu verwenden. Bevorzugt werden jedoch natürliche Wachse. Der Baumwollstoff ist zu einer Röhre gerollt, derart, dass beim Rollen wenigstens eine Lage des Stoffes überlappend oder mehrere Lagen des Stoffes übereinanderliegen.

Diese Röhre wird mit einem Ende auf den äußeren Gehörgang aufgesetzt, und das andere Ende wird angezündet, so dass eine offene Flamme entsteht.

Die Ohrkerzen wirken rein physikalisch. Ein leichter Unterdruck (Kamineffekt) und die durch die Bewegung der Flamme hervorgerufenen Vibrationswellen der Luft in der Kerze wirken wie eine sanfte Trommelfellmassage. Dies führt zu einem intensiven Gefühl angenehmer Wärme und einem als befreiend empfundenen Druckausgleich im Ohr-, Stirn- und Nebenhöhlenbereich.

Die Wachsmenge in dem Baumwollstoff ist auf den Verbrennungsvorgang abgestimmt. Das heißt, dass im optimalen Fall die Ohrkerze möglichst rückstandsfrei verbrennen soll.

Beim Abbrennen bleibt eine Aschefahne erhalten, die zum Schluss in der Regel abknickt.

Im Inneren der Ohrkerze entsteht ein Gasgemisch. Das Gasgemisch kühlt in der Ohrkerze ab, fällt nach unten, kondensiert und würde ohne Rückhalteelement ungehindert in den äußeren Gehörgang gelangen.

Beim Abbrennen der Ohrkerze schmilzt darüber hinaus das Wachs. In seltenen Fällen kann es vorkommen, dass, wenn das Wachs in zu großer Menge vorhanden ist, auch das Wachs in der Ohrkerze nach unten fließt und ohne Rückhalteelement in den äußeren Gehörgang gelangen würde.

Die im Stand der Technik (DE 10 2006 004 138 A1) beschriebene Ohrkerze mit Rückhalteelement weist den Nachteil auf, dass das dort beschriebene Rückhalteelement das entstehende Kondensat und eventuell abfließendes Wachs entweder nicht ausreichend zurückhält, je nach dem, aus welchem Material das Rückhalteelement besteht, beispielsweise wenn es aus einem Fliegengitter oder einem feinmaschigen Draht besteht. Besteht das Rückhalteelement aus einem Filterpapier oder einem Filterstoff, setzt es sich zu leicht zu, so dass die Luftdurchlässigkeit nicht mehr gewährleistet ist.

Zum Stand der Technik (EP 0 908 161 A1) gehört ein Sicherheitszubehör für Stoff- und Wachskonen, welche bei der Ohrreinigung eingesetzt werden.

Diese zum Stand der Technik gehörende Ohrkerze weist ein Rückhalteelement auf, welches zwar an der Innenseite der Ohrkerze herablaufendes Wachs und Kondensat auffängt. Herabtropfendes Wachs kann jedoch durch das Filterelement mittig durchtropfen oder -laufen.

Weiterhin gehört zum Stand der Technik (FR 2 768 924 A1) ein Filter für eine Ohrkerze. Dieser zum Stand der Technik gehörende Filter besteht aus einem gewölbt ausgebildeten Gitternetz. Durch dieses Gitternetz kann Kondensat oder Wachs, das an der Innenwand der Ohrkerze herunterläuft, fast ungehindert in das Ohr gelangen.

Das der Erfindung zugrunde liegende technische Problem besteht darin, eine Ohrkerze mit einem Rückhalteelement anzugeben, welches Kondensat und eventuell abfließendes Bienenwachs zuverlässig am Weiterfließen hindert und darüber hinaus auch bei größeren Kondensat- und Wachsmengen eine Luftdurchlässigkeit des Rückhalteelementes gewährleistet.

Dieses technische Problem wird durch eine Ohrkerze mit einem luftdurchlässigen Rückhalteelement mit den Merkmalen gemäß Anspruch 1 gelöst.

Die erfindungsgemäße Ohrkerze mit einem luftdurchlässigen Rückhalteelement, welches wenigstens teilweise innerhalb der Ohrkerze angeordnet ist, wobei das Rückhalteelement eine Wand und einen Innenraum aufweist, zeichnet sich dadurch aus, dass an der Wand wenigstens zwei in den Innenraum gerichtete Lamellen angeordnet sind, und dass die wenigstens zwei Lamellen in axialer Richtung sich überlappend ausgebildet sind.

Durch die erfindungsgemäße Ausführungsform des Rückhalteelementes in der Ohrkerze ist gewährleistet, dass Kondensat und eventuell abfließendes Wachs sehr zuverlässig in dem Rückhalteelement an dem Weiterfließen gehindert wird. Das erfindungsgemäße Rückhalteelement setzt sich auch bei größeren Kondensat- und Wachsmengen nicht zu, so dass die Luftdurchlässigkeit gewährleistet ist.

Die Lamellen sind vorteilhaft mit ihrer der Wand zugewandten Seite bündig an der Wand anliegend ausgebildet. Sie liegen mindestens halbkreisförmig an der Wand des Rückhalteelementes an. Außerdem ragen sie über eine Mittellinie des Rückhalteelementes. Die Lamellen decken mehr als die Hälfte der Öffnung des Rückhalteelementes ab.

Die wenigstens zwei Lamellen sind in axialer Richtung sich überlappend ausgebildet. Dadurch, dass die wenigstens zwei Lamellen sich gegenüberliegend in dem Innenraum angeordnet sind, ist das Rückhalteelement in axialer Richtung gesehen optisch dicht, so dass sogar herabtropfendes Kondensat oder herabtropfendes Wachs von den Lamellen aufgefangen wird.

Gemäß einer besonders bevorzugten Ausführungsform der Erfindung sind wenigstens drei Lamellen in dem Rückhalteelement angeordnet, wobei wenigstens eine Lamelle den anderen Lamellen gegenüberliegend angeordnet und diese in axialer Richtung überlappend ausgebildet ist. Auch mit drei Lamellen ist das Rückhalteelement in axialer Richtung gesehen optisch dicht ausgebildet, damit selbst herabtropfendes Wachs oder Kondensat nicht in den äußeren Gehörgang gelangen kann, sondern von den Lamellen aufgefangen wird.

Gemäß einer weiteren vorteilhaften Ausführungsform der Erfindung weist das Rückhalteelement in einer Mantelfläche wenigstens eine Öffnung auf. Hierdurch ist das erfindungsgemäße Rückhalteelement, wenn es als Spritzgusselement ausgebildet ist, einfacher herzustellen.

Gemäß einer weiteren vorteilhaften Ausführungsform der Erfindung ist die wenigstens eine Öffnung im Bereich der wenigstens einen Lamelle angeordnet. Hierdurch wird aus dem Material, welches sonst im Bereich der Öffnung die Wand des Rückhalteelementes bilden würde, für die Ausbildung der Lamelle verwendet wird.

Die wenigstens zwei Lamellen sind vorteilhaft in einem Winkel von 30° bis 60° in dem Rückhalteelement angeordnet. Gemäß einer besonders bevorzugten Ausführungsform der Erfindung sind die wenigstens zwei Lamellen in einem Winkel von 45° in dem Rückhalteelement angeordnet. Hierdurch ist gewährleistet, dass zum einen die Lamellen sich derart überlappen, dass das Rückhalteelement in axialer Richtung gesehen optisch dicht ist, so dass kein Kondensat oder Wachs in den äußeren Gehörgang gelangen kann, und dass andererseits ein ausreichender Luftdurchlass in dem Rückhalteelement gegeben ist.

Eine Weiterbildung der Erfindung sieht vor, dass das Rückhalteelement einen Stutzen zum Ansatz auf den äußeren Gehörgang (Ansatzstutzen) aufweist, der einen zur Ohrkerze hin sich erweiternden Durchmesser aufweist. Hierdurch ist gewährleistet, dass das Rückhalteelement mit dem Ansatzstutzen leicht und verletzungsfrei auf den äußeren Gehörgang aufgesetzt werden kann. Durch eine Weiterbildung mit abgerundeten Außenflächen des Ansatzstutzens ist gewährleistet, dass keine scharfen Kanten vorhanden sind und damit die Haut nicht verletzt wird. Darüber hinaus ist die Haptik der Ohrkerze deutlich verbessert.

In Richtung der Ohrkerze weist der Ansatzstutzen vorteilhaft einen Überstand auf, der der Dicke des Materials der Ohrkerze entspricht. Hierdurch schließt der Ansatzstutzen, der teilweise in der Ohrkerze angeordnet ist und zum Teil über die Ohrkerze übersteht, bündig mit einer Hülse der Ohrkerze ab. Wie schon ausgeführt, besteht die Ohrkerze beispielsweise aus einem mit Bienenwachs getränkten Baumwollstoff. Dieser Baumwollstoff wird zu der Ohrkerze gerollt, so dass die Ohrkerze innen einen Hohlraum aufweist. Die Hülse der Ohrkerze besteht damit aus dem ein- oder mehrlagigen Baumwollstoff, der mit Bienenwachs getränkt ist.

Wie schon ausgeführt, steht der Ansatzstutzen vorteilhaft in axialer Richtung über die Ohrkerze über. Hierdurch ist gewährleistet, dass die Ohrkerze mit dem Ansatzstutzen des Rückhalteelementes auf den äußeren Gehörgang aufgesetzt werden kann, ohne dass eine Verletzungsgefahr der Haut besteht.

Das Rückhalteelement ist vorteilhaft zylinderförmig oder kegelabschnittsförmig ausgebildet. Hierdurch ist es möglich, das Rückhalteelement in die vorgefertigte Ohrkerze in einfacher Art und Weise einzuschieben.

Das Rückhalteelement ist vorteilhaft in der Ohrkerze ohne zusätzliche Klebemittel klemmend gehalten.

Das Rückhalteelement besteht vorteilhaft aus Kunststoff, Glas, Keramik, Holz und Metall. Eine besonders bevorzugte Ausführungsform sieht vor, das Rückhalteelement aus Kunststoff auszubilden. Besonders bevorzugt wird als Kunststoff Polypropylen (PP) verwendet. Es ist jedoch auch möglich, Polypropylencarbonat (PPC) oder Polyethylen (PE) zu verwenden. Es ist ebenfalls möglich, ein Gemisch vorzusehen.

Weitere Merkmale und Vorteile der Erfindung ergeben sich anhand der zugehörigen zeichnung, in der mehrere Ausführungsbeispiele eines erfindungsgemäßen Rückhalteelementes einer Ohrkerze nur beispielhaft dargestellt sind. In der Zeichnung zeigen:
- Fig. 1: einen Längsschnitt durch ein Rückhalteelement mit drei Lamellen;
- Fig. 2: eine Ansicht in Richtung des Pfeiles II der Fig. 1 (ohne das Teil 21);
- Fig. 3: einen Längsschnitt durch ein Rückhalteelement mit zwei Lamellen;
- Fig. 4: ein Rückhalteelement mit zwei Elementen in perspektiver Ansicht, längs aufgeschnitten;
- Fig. 5: einen Längsschnitt durch eine Ohrkerze mit einem Rückhalteelement.

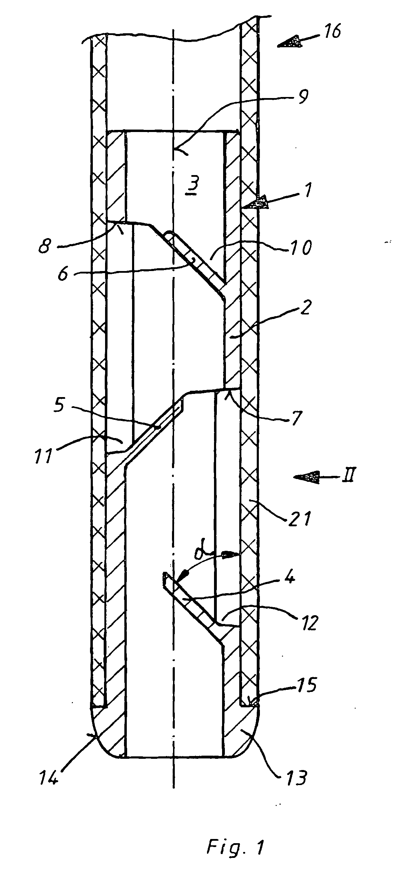

Fig. 1 zeigt ein Rückhalteelement 1, welches eine Wand 2 und einen Innenraum 3 aufweist. In dem Innenraum sind drei Lamellen 4, 5, 6 angeordnet, die mit einem Winkel α von 45° angeordnet sind.

Im Bereich der Lamellen 4, 5 sind Öffnungen 7, 8 in dem Rückhalteelement angeordnet.

Wie man an der eingezeichneten Mittellinie 9 erkennt, ragen die Lamellen 4, 5, 6 jeweils über die Mittellinie 9 hinaus, das heißt die Lamellen 4, 5, 6 überlappen sich in axialer Richtung gesehen, so dass das Rückhalteelement in axialer Richtung gesehen optisch dicht ausgebildet ist. Hierdurch ist gewährleistet, dass Kondensat und eventuell abfließendes Wachs 100%ig von dem Rückhalteelement aufgefangen wird.

Das abfließende Wachs sammelt sich insbesondere in den Vertiefungen 10, 11, 12. Das bedeutet wiederum, dass der Luftdurchlass in dem Rückhalteelement von dem sich sammelnden Kondensat und Wachs nicht beeinträchtigt wird.

Das Rückhalteelement weist einen Ansatzstutzen 13 auf, der eine abgerundete Außenfläche 14 aufweist. Darüber hinaus weist der Ansatzstutzen 13 einen Überstand 15 auf, derart, dass das Rückhalteelement 1 mit einer Hülse 21 einer Ohrkerze 16 bündig abschließt. Das Rückhalteelement 1 ist teilweise in der Hülse 21 der Ohrkerze 16 angeordnet und steht mit dem Ansatzstutzen 13 über.

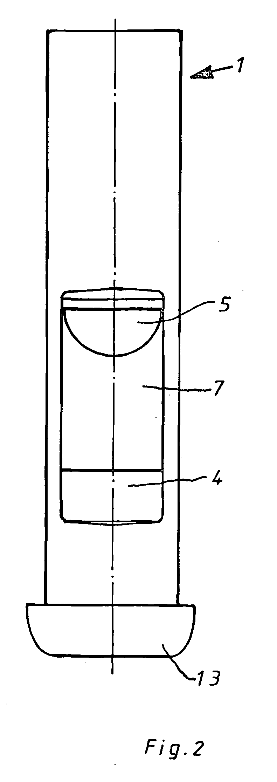

Fig. 2 zeigt das Rückhalteelement 1 mit dem Ansatzstutzen 13 und der Öffnung 7 sowie den Lamellen 4 und 5. Der besseren Übersicht wegen ist die Hülse 21 in Fig. 2 nicht dargestellt.

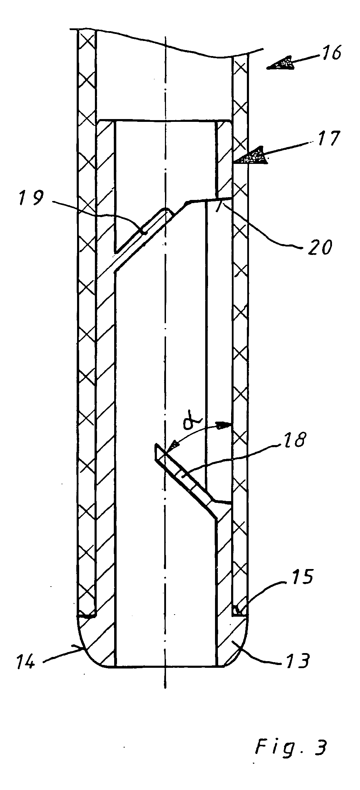

Fig. 3 zeigt ein Rückhalteelement 17, welches zwei Lamellen 18, 19 aufweist. Das Rückhalteelement 17 ist in der Ohrkerze 16 angeordnet. Auch die Lamellen 18, 19 überlappen sich, so dass abfließendes Kondensat und sogar herabtropfendes Kondensat und Wachs nicht durch das Rückhalteelement hindurch in Richtung Ohr (nicht dargestellt) gelangen kann.

Das Rückhalteelement 17 weist eine Öffnung 20 auf, welche im Bereich der Lamellen 18, 19 angeordnet ist.

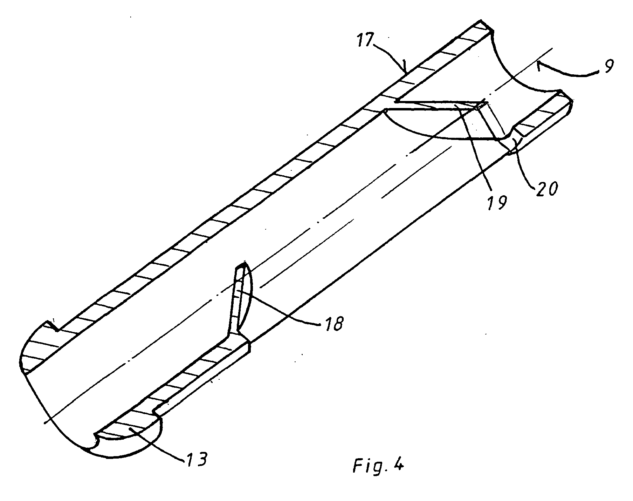

Fig. 4 zeigt das Rückhalteelement 17 mit den Lamellen 18, 19 sowie der Öffnung 20.

In den Fig. 3 und 4 ist ebenfalls der Ansatzstutzen 13 dargestellt, der die gleiche Ausbildung und Funktion wie bei der Ausführungsform gemäß den Fig. 1 und 2 aufweist.

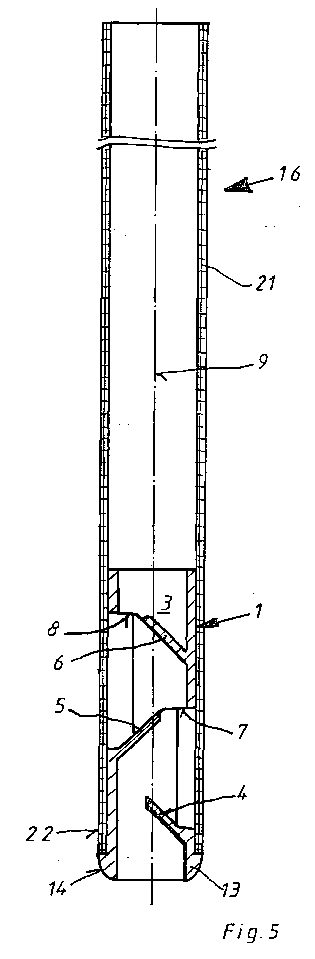

Fig. 5 zeigt die Ohrkerze 16 mit der Hülse 21, die aus einem mit Bienenwachs getränkten Baumwollstoff besteht. Im ohrseitigen Teil 22 der Ohrkerze 16 ist das Rückhalteelement 1 angeordnet. Das Rückhalteelement 1 weist die Lamellen 4, 5, 6 auf sowie die Öffnungen 7, 8, die durch die Hülse 21 verschlossen sind.

Das Rückhalteelement 1 weist darüber hinaus den Ansatzstutzen 13 auf, damit die Ohrkerze verletzungsfrei mit deutlich verbesserter Haptik platziert werden kann.

### Bezugszahlen

- 1: Rückhalteelement
- 2: Wand
- 3: Innenraum
- 4: Lamelle
- 5: Lamelle
- 6: Lamelle
- 7: Öffnung
- 8: Öffnung
- 9: Mittellinie
- 10: Vertiefung
- 11: Vertiefung
- 12: Vertiefung
- 13: Ansatzstutzen
- 14: abgerundete Außenfläche
- 15: Überstand
- 16: Ohrkerze
- 17: Rückhalteelement
- 18: Lamelle
- 19: Lamelle
- 20: Öffnung
- 21: Hülse einer Ohrkerze
- 22: ohrseitiger Teil der Ohrkerze 16

## Patentansprüche

1. Ohrkerze mit einem luftdurchlässigen Rückhalteelement, welches wenigstens teilweise innerhalb der Ohrkerze angeordnet ist, wobei das Rückhalteelement eine Wand und einen Innenraum aufweist,
**dadurch gekennzeichnet, dass** an der Wand (2) wenigstens zwei in den Innenraum (3) gerichtete Lamellen (4, 5, 6; 18, 19) angeordnet sind, dass die wenigstens zwei Lamellen sich gegenüberliegend in dem Innenraum (3) angeordnet sind, und dass die wenigstens zwei Lamellen (4, 5, 6; 18, 19) in axialer Richtung sich überlappend ausgebildet sind.

2. Ohrkerze nach Anspruch 1, **dadurch gekennzeichnet, dass** wenigstens drei Lamellen (4, 5, 6) in dem Rückhalteelement (1) angeordnet sind, und dass wenigstens eine Lamelle (5) den anderen Lamellen (4, 6) gegenüberliegend angeordnet ist und diese Lamellen (4, 6) in axialer Richtung überlappend ausgebildet sind.

3. Ohrkerze nach Anspruch 1 oder 2, **dadurch gekennzeichnet, dass** das Rückhalteelement (1) in der Wand (2) wenigstens eine Öffnung (7, 8, 20) aufweist.

4. Ohrkerze nach Anspruch 3, **dadurch gekennzeichnet, dass** die wenigstens eine Öffnung (7, 8, 20) im Bereich wenigstens einer Lamelle (4, 5, 18) angeordnet ist.

5. Ohrkerze nach einem der vorhergehenden Ansprüche, **dadurch gekennzeichnet, dass** die wenigstens zwei Lamellen (4, 5, 6; 18, 19) in einem Winkel von 30° bis 60° in dem Rückhalteelement (1) angeordnet sind.

6. Ohrkerze nach Anspruch 5, **dadurch gekennzeichnet, dass** die wenigstens zwei Lamellen (4, 5, 6; 18, 19) in einem Winkel von 45° in dem Rückhalteelement (1) angeordnet sind.

7. Ohrkerze nach einem der vorhergehenden Ansprüche, **dadurch gekennzeichnet, dass** die wenigstens zwei Lamellen (4, 5, 6; 18, 19) bündig und wenigstens halbkreisförmig an dem Rückhalteelement angeordnet sind.

8. Ohrkerze nach einem der vorhergehenden Ansprüche, **dadurch gekennzeichnet, dass** das Rückhalteelement (1) einen Ansatzstutzen (13) aufweist, der einen zur Ohrkerze (16) hin sich erweiterten Durchmesser aufweist.

9. Ohrkerze nach Anspruch 8 **dadurch gekennzeichnet, dass** der Ansatzstutzen (13) eine abgerundete Außenfläche (14) aufweist.

10. Ohrkerze nach einem der Ansprüche 8 oder 9, **dadurch gekennzeichnet, dass** der Ansatzstutzen (13) in Richtung Ohrkerze (16) einen Überstand (15) aufweist, der der Dicke einer Hülse (21) der Ohrkerze (16) entspricht.

11. Ohrkerze nach einem der Ansprüche 8 bis 10, **dadurch gekennzeichnet, dass** der Ansatzstutzen (13) in axialer Richtung über die Ohrkerze (16) überstehend ausgebildet ist.

12. Ohrkerze nach einem der vorhergehenden Ansprüche, **dadurch gekennzeichnet, dass** das Rückhalteelement (1) zylinderförmig oder kegelabschnittsförmig ausgebildet ist.

13. Ohrkerze nach einem der vorhergehenden Ansprüche, **dadurch gekennzeichnet, dass** das Rückhalteelement (1) aus Kunststoff, Glas, Keramik, Holz oder Metall gebildet ist.

14. Ohrkerze nach einem der vorhergehenden Ansprüche, **dadurch gekennzeichnet, dass** das Rückhalteelement (1) aus Polypropylen (PP), Polypropylencarbonat (PPC) und/oder Polyethylen (PE) gebildet ist.

## Claims

1. Ear candle with an air-permeable retaining member disposed at least partially within the ear candle, wherein the retaining member comprises a wall and an interior space,
**characterized in that** at least two lamellas (4, 5, 6; 18, 19) directed into the interior space (3) are disposed on the wall (2), that the at least two lamellas are disposed opposite from each other in the interior space (3), and that the at least two lamellas (4, 5, 6; 18, 19) are configured so as to overlap in the axial direction.

2. Ear candle according to claim 1, **characterized in that** at least three lamellas (4, 5, 6) are disposed in the retaining member (1), and that at least one lamella (5) is disposed opposite the other lamellas (4, 6) and configured so as to overlap these lamellas (4, 6) in the axial direction.

3. Ear candle according to claim 1 or 2, **characterized in that** the retaining member (1) has at least one opening (7, 8, 20) in the wall (2).

4. Ear candle according to claim 3, **characterized in that** the at least one opening (7, 8, 20) is disposed in the area of at least one lamella (4, 5, 18).

5. Ear candle according to any one of the preceding claims, **characterized in that** the at least two lamellas (4, 5, 6; 18, 19) are disposed at an angle of 30° to 60° in the retaining member (1).

6. Ear candle according to claim 5, **characterized in that** the at least two lamellas (4, 5, 6; 18, 19) are disposed at an angle of 45° in the retaining member (1).

7. Ear candle according to any one of the preceding claims, **characterized in that** the at least two lamellas (4, 5, 6; 18, 19) are disposed flush with and at least in a semi-circular shape on the retaining member.

8. Ear candle according to any one of the preceding claims, **characterized in that** the retaining member (1) has a fitting nozzle (13) having a diameter widening towards the ear candle (16).

9. Ear candle according to claim 8, **characterized in that** the fitting nozzle (13) comprises a rounded external surface (14).

10. Ear candle according to any one of the claims 8 or 9, **characterized in that** the fitting nozzle (13) has a projecting portion (15) in the direction of the ear candle (16), which projecting portion corresponds to the thickness of a sleeve (21) of the ear candle (16).

11. Ear candle according to any one of the claims 8 to 10, **characterized in that** the fitting nozzle (13) is configured to protrude over the ear candle (16) in the axial direction.

12. Ear candle according to any one of the preceding claims, **characterized in that** the retaining member (1) is configured in the shape of a cylinder or a section of a cone.

13. Ear candle according to any one of the preceding claims, **characterized in that** the retaining member (1) is formed from plastic, glass, ceramics, wood or metal.

14. Ear candle according to any one of the preceding claims, **characterized in that** the retaining member (1) is formed from polypropylene (PP), polypropylene carbonate (PPC) and/or polyethylene (PE).

## Revendications

1. Bougie d'oreille comprenant un élément de retenue perméable à l'air qui est disposé au moins en partie à l'intérieur de la bougie d'oreille, ledit élément de retenue présentant une paroi et un volume intérieur,
**caractérisée par le fait que** sur ladite paroi (2) sont disposées au moins deux lamelles (4, 5, 6 ; 18, 19) dirigées vers ledit volume intérieur (3), que lesdites au moins deux lamelles sont disposées en regard l'une de l'autre dans le volume intérieur (3) et que lesdites au moins deux lamelles (4, 5, 6 ; 18, 19) sont réalisées de manière à se chevaucher dans la direction axiale.

2. Bougie d'oreille selon la revendication 1, **caractérisée par le fait que** trois lamelles (4, 5, 6) au moins sont disposées dans ledit élément de retenue (1) et qu'au moins une lamelle (5) est disposée en regard des autres lamelles (4, 6) et que ces lamelles (4, 6) sont réalisées de manière à se chevaucher dans la direction axiale.

3. Bougie d'oreille selon la revendication 1 ou 2, **caractérisée par le fait que** ledit élément de retenue (1) présente au moins un orifice (7, 8, 20) dans ladite paroi (2).

4. Bougie d'oreille selon la revendication 3, **caractérisée par le fait que** ledit au moins un orifice (7, 8, 20) est disposé au niveau d'au moins une lamelle (4, 5, 18).

5. Bougie d'oreille selon l'une quelconque des revendications précédentes, **caractérisée par le fait que** lesdites au moins deux lamelles (4, 5, 6 ; 18, 19) sont disposées à l'intérieur dudit élément de retenue (1) à un angle compris entre 30° et 60°.

6. Bougie d'oreille selon la revendication 5, **caractérisée par le fait que** lesdites au moins deux lamelles (4, 5, 6 ; 18, 19) sont disposées à l'intérieur dudit élément de retenue (1) à un angle de 45°.

7. Bougie d'oreille selon l'une quelconque des revendications précédentes, **caractérisée par le fait que** lesdites au moins deux lamelles (4, 5, 6 ; 18, 19) sont à fleur et disposées au moins en demi-cercle sur ledit élément de retenue.

8. Bougie d'oreille selon l'une quelconque des revendications précédentes, **caractérisée par le fait que** ledit élément de retenue (1) comprend une tubulure de raccord (13) qui présente un diamètre s'élargissant vers la bougie d'oreille (16).

9. Bougie d'oreille selon la revendication 8, **caractérisée par le fait que** ladite tubulure de raccord (13) présente une surface extérieure (14) arrondie.

10. Bougie d'oreille selon l'une quelconque des revendications 8 ou 9, **caractérisée par le fait que** ladite tubulure de raccord (13) présente une projection (15) en direction de la bougie d'oreille (16), qui correspond à l'épaisseur d'une douille (21) de ladite bougie d'oreille (16).

11. Bougie d'oreille selon l'une quelconque des revendications 8 à 10, **caractérisée par le fait que** ladite tubulure de raccord (13) est réalisée de manière à dépasser la bougie d'oreille (16) dans la direction axiale.

12. Bougie d'oreille selon l'une quelconque des revendications précédentes, **caractérisée par le fait que** ledit élément de retenue (1) est réalisé en forme de cylindre ou en forme de section conique.

13. Bougie d'oreille selon l'une quelconque des revendications précédentes, **caractérisée par le fait que** ledit élément de retenue (1) est réalisé en matière plastique, en verre, en céramique, en bois ou en métal.

14. Bougie d'oreille selon l'une quelconque des revendications précédentes, **caractérisée par le fait que** ledit élément de retenue (1) est réalisé en polypropylène (PP), en polypropylène carbonate (PPC) et/ou en polyéthylène (PE).
